# EUROPÄISCHE PATENTSCHRIFT

(11) **EP 1 120 388 B1**
(45) Veröffentlichungstag und Bekanntmachung des Hinweises auf die Patenterteilung: **17.03.2004**
(21) Anmeldenummer: 01105436.8
(22) Anmeldetag: 06.08.1997
(51) Int. Cl.: C05G 3/08, C05G 3/00, C05F 3/00, C07D 231/16, C07D 321/12

(54) **Verwendung von Pyrazolderivaten als Nitrifikationsinhibitoren**
Use of pyrazol derivatives as nitrification inhibitors
Utilisation de dérivés de pyrazole comme inhibiteurs de nitrification

(30) Priorität: 06.08.1996 DE 19631764
(43) Veröffentlichungstag der Anmeldung: 01.08.2001
(62) Teilanmeldung aus: 97941915.7
(73) Patentinhaber: BASF AKTIENGESELLSCHAFT, 67056 Ludwigshafen (DE)
(72) Erfinder: Barth, Thomas, Dr., 67071 Ludwigshafen (DE); Rieber, Norbert, Dr., 68259 Mannheim (DE); Gold, Randall Evan, Dr., Research Triangle Park, NC 27709 (US); Dressel, Jürgen, Dr., 67141 Neuhofen (DE); Erhardt, Klaus, Dr., 69181 Leimen (DE); Rittinger, Stefan, Dr., 68199 Mannheim (DE); Horchler von Locquenghien, Klaus, Dr., 67117 Limburgerhof (DE); Leibold, Edgar, Dr., 67316 Carlsberg (DE)
(74) Vertreter: Isenbruck, Günter, Dr.

(56) Entgegenhaltungen:
- EP-A- 0 166 420
- EP-A- 0 529 473
- DD-A- 222 471
- DD-A- 273 829
- DE-A- 4 405 393
- DE-A- 4 446 194
- US-A- 3 635 690
- US-A- 4 523 940
- US-A- 4 975 107
- CHEMICAL ABSTRACTS, vol. 112, no. 13, 26. März 1990 (1990-03-26) Columbus, Ohio, US; abstract no. 117849f, F.KH. KHAZIEV ET AL.: "Composition for inhibiting nitrification of urea in soil." Seite 650; XP000181047 & SU 1 477 726 A (BASHKIR INSTITUTE OF CHEMISTRY ET AL.) 7. Mai 1989 (1989-05-07)
- CHEMICAL ABSTRACTS, vol. 113, no. 17, 22. Oktober 1990 (1990-10-22) Columbus, Ohio, US; abstract no. 151358a, H.J. KLASSE ET AL.: "Effect of ammonium thiosulfate on the nitrification-inhibiting activity of dicyandiamide." Seite 686; XP000182729 & VDLUFA-SCHRIFTENR., Bd. 30, 1990, Seite 181-186
- CHEMICAL ABSTRACTS, vol. 118, no. 15, 12. April 1993 (1993-04-12) Columbus, Ohio, US; abstract no. 146794p, V.D. PARKHOMENKO ET AL.: "Kinetics of the thermal decomposition of nitrification inhibitors based on 3(5)-methylpyrazole derivatives." Seite 770; XP000354133 & ZH. PRIKL. KHIM. (S.-PETERBURG), Bd. 65, Nr. 6, 1992, Seiten 1362-1367,

## Beschreibung

Die Erfindung betrifft die Verwendung von ausgewählten Pyrazolderivaten als Nitrifikationsinhibitoren in Mineraldüngemitteln sowie als Stabilisatoren von Gülle oder flüssigen Düngeformulierungen, sowie die entsprechenden behandelten Mineraldüngemittel an sich. Darüber hinaus betrifft die vorliegende Erfindung Pyrazolderivate, die als Nitrifikationsinhibitoren in Mineraldüngemitteln sowie als Stabilisatoren von Gülle oder flüssigen Düngeformulierungen verwendet werden können.

Um Pflanzen in der Landwirtschaft den von ihnen benötigten Stickstoff zur Verfügung zu stellen, werden im wesentlichen Ammoniumverbindungen als Düngemittel eingesetzt.

Ammoniumverbindungen werden im Boden in relativ kurzer Zeit mikrobiell zu Nitrat umgesetzt (Nitrifikation). Nitrat kann jedoch aus dem Boden ausgewaschen werden. Der ausgewaschene Anteil steht dabei für die Pflanzenernährung nicht mehr zur Verfügung, so daß aus diesem Grund eine schnelle Nitrifikation unerwünscht ist. Zur besseren Ausnutzung des Düngers werden deshalb dem Dünger Nitrifikationsinhibitoren zugesetzt. Eine bekannte Gruppe von Nitrifikationsinhibitoren sind Pyrazolverbindungen.

Ein Problem bei der Verwendung von Pyrazolverbindungen als Nitrifikationsinhibitoren ist deren hohe Flüchtigkeit. Bei der Lagerung von Pyrazolverbindungen enthaltenden Düngerzubereitungen tritt somit ein kontinuierlicher Verlust an Wirkstoff durch Verdampfung auf. Deshalb müssen die Pyrazolverbindungen durch geeignete Maßnahmen in einer nichtflüchtigen Form formuliert werden.

Es wurde versucht, durch Neutralisierung der Pyrazolverbindungen mit Mineralsäuren, wie Phosphorsäure oder Salzsäure, ihre Flüchtigkeit zu vermindern. In der DE-A-4 128 828 ist die Verwendung von Nitraten und Phosphaten des 3-Methylpyrazols zur Beschichtung von Düngern beschrieben. Die US 3 635 690 beschreibt ebenfalls die Stabilisierung von Pyrazolderivaten durch Mineralsäuren, wie Salzsäure, Schwefelsäure, Salpetersäure und Phosphorsäure. Diese sauren Salze der Pyrazolverbindungen sind jedoch immer noch hydrolyseanfällig und aus diesem Grund nicht für alle Anwendungen einsetzbar.

Aufgabe der vorliegenden Erfindung ist die Bereitstellung von neuen Nitrifikationsinhibitoren.

Die Aufgabe wird gelöst durch Verwendung von 3,4-Dimethylpyrazol oder einem Phosphorsäure-Additionssalz davon oder einem Phosphorsäureadditionssalz von 4-Chlor-3-methylpyrazol als Nitrifikationsinhibitoren.

Die Pyrazolverbindungen können in der basischen Form eingesetzt werden, wie auch in Form von Säureadditionssalzen mit anorganischen Mineralsäuren und organischen Säuren. Beispiele anorganischer Mineralsäuren sind Salzsäure, Phosphorsäure, Schwefelsäure, vorzugsweise Phosphorsäure. Beispiele organischer Säuren sind Ameisensäure, Essigsäure, wie auch Fettsäuren.
Beispiele entsprechender Salze sind die Hydrochloride und Phosphorsäure-Additionssalze.

Die Pyrazolverbindungen können einzeln oder in Form von Gemischen eingesetzt werden.

Die Pyrazolverbindungen sind 3,4-Dimethylpyrazol, sowie die Phosphorsäure-Additionssalze von 3,4-Dimethylpyrazol und 4-Chlor-3-methylpyrazol.

Durch Verwendung der Säureadditionssalze der Pyrazolverbindungen kann die Flüchtigkeit der Verbindungen weiter herabgesetzt werden. Somit werden vorteilhaft Säureadditionssalze der Pyrazolverbindungen eingesetzt.

Die Herstellung der erfindungsgemäß verwendeten Pyrazolverbindungen ist bekannt. Sie ist beispielsweise beschrieben in EP-A-0 474 037, DE-A 3 840 342, EP-A-0 467 707. Zur Herstellung von 3,4-Dimethylpyrazol kann zudem auf Noyce et al., Jour. of Org. Chem. 20, 1955, 1681 bis 1682 verwiesen werden.

Die Säureadditionssalze der Pyrazolverbindungen erhält man durch Umsetzung der Pyrazole mit einem Äquivalent an entsprechender Säure.

### Behandelte Mineraldüngemittel

Erfindungsgemäß werden Mineraldüngemittel eingesetzt. Dies sind Ammonium oder Harnstoff enthaltende Düngemittel. Beispiele derartiger ammoniumhaltiger Düngemittel sind NPK-Düngemittel, Kalkammonsalpeter, Ammonsulfatsalpeter, Ammoniumsulfat oder Ammoniumphosphat.

Die erfindungsgemäßen Mineraldüngemittel können in Pulverform oder in Granulatform vorliegen.

Das erfindungsgemäße Mineraldüngemittel enthält mindestens einen Nitrifikationsinhibitor. Dabei kann das Mineraldüngemittel im Gemisch mit dem Nitrifikationsinhibitor vorliegen. Der Nitrifikationsinhibitor kann auch auf der Oberfläche des Mineraldüngemittels vorliegen.

Ferner betrifft die vorliegende Erfindung Mineraldüngemittel, enthaltend eine Verbindung, wie sie vorstehend definiert ist.

Ferner betrifft die vorliegende Erfindung ein Düngeverfahren, wobei man Mineraldüngemittel, wie sie im Rahmen der vorliegenden Anmeldung definiert sind, auf den Ackerboden ausbringt.

Dabei werden die beschriebenen Nitrifikationsinhibitoren oder Mineraldüngemittel gemäß einer Ausführungsform der Erfindung nicht zur Behandlung von Böden, die für Mais-, Baumwolle-, Weizen-, Reis-, Gerste- und/oder Zuckerrübenkulturen vorgesehen sind, oder der entsprechenden Kulturen eingesetzt.

Ferner können die beschriebenen Verbindungen zur Stabilisierung von Gülle oder flüssigen Düngeformulierungen, wie Ammoniumnitrat-Harnstoff-Lösungen oder flüssigem Ammoniak, verwendet werden.

Ferner betrifft die Erfindung die Phosphorsäure-Additionssalze von 3,4-Dimethylpyrazol und 4-Chlor-3-methylpyrazol an sich, sowie Gemische dieser Verbindungen, die ebenfalls als Nitrifikationsinhibitoren verwendet werden können.

Nachstehend wird die Erfindung anhand von Beispielen näher beschrieben.

### Beispiele

### Herstellung von 3,4-Dimethylpyrazoliumdihy drogenphosphat

96 g (1,0 mol) 3,4-Dimethylpyrazol wurden in 115 g (1,0 mol) Phosphorsäure (85 %) bei Raumtemperatur gelöst. Das in der Phosphorsäure enthaltene Wasser wurde abgedampft. Nach einigen Stunden kristallisierte die Titelverbindung aus dem zunächst vorliegenden Öl (Ausbeute 98 %).

### Herstellung von 4-Chlor-3-methylpyrazoliumdihydrogenphosphat

116 g (1,0 mol) 4-Chlor-3-methylpyrazol wurden in 115 g (1,0 mol) Phosphorsäure (85%) bei Raumtemperatur gelöst. Das in der Phosphorsäure enthaltene Wasser wurde abgedampft. Nach einigen Stunden kristallisierte die Titelverbindung aus dem zunächst vorliegenden Öl (Ausbeute 98 %).

### Nachweis der biologischen Wirkung der Nitrifikationsinhibitoren

### - Feldversuche -

Die biologische Wirksamkeit von 4 Chlor-3-methylpyrazol (4Cl-3MP) (Referenz) und 3,4-Dimethylpyrazol (3,4-DMP) im Vergleich zu DCD und der Kontrolle wurde in mehreren Feldversuchen in unterschiedlichen Umwelten an Hand der Merkmale "Nitratgehalt in der Stengelbasis", "NO₃- und NH₄-N-Gehalt im Boden" sowie "Kornertrag" geprüft.

Für die Anlage, Beprobung, Beerntung und Auswertung der Feldversuche sind die im landwirtschaftlichen Versuchswesen üblichen Verfahren angewendet worden.

Die Analyse der Pflanzen- und Bodenproben erfolgte nach Standardmethoden. Die übrigen produktionstechnischen Maßnahmen, wie der Pflanzenschutz, entsprachen der guten landwirtschaftlichen Praxis und wurden einheitlich durchgeführt.

Ein biologisch wirksamer Nitrifikationsinhibitor zeichnet sich vorzugsweise dadurch aus, daß er in einem Zeitraum von bis zu 8 Wochen nach der Applikation gegenüber der Kontrolle (hier Trägerdünger Ammonsulfatsalpeter ohne Nitrifikationshemmstoff) im Boden niedrigere Gehalte an NO₃-N und höhere Gehalte an NH₄-N aufweist (Tab. 1). Als Folge dieser Bedingungen ist die Nitrataufnahme von Pflanzen reduziert (vgl. NO₃-Gehalt in der Stengelbasis von Rapspflanzen, Tab. 2) und häufig der Ertrag erhöht (vgl. Kornertrag von Winterweizen, Tab. 3. Tabelle 4 gibt die Standortbescbreibung der Feldversuche wieder.

In der folgenden Tabelle 1 sind die Ergebnisse zusammengefaßt. Es wird deutlich, daß alle drei Nitrifikationsinhibitoren eine gute biologische Wirksamkeit im Vergleich zur Kontrolle aufweisen. 4Cl-3MP (Referenz) und 3,4-DMP zeichnen sich durch eine ebenso gute und z. T. bessere Wirksamkeit gegenüber DCD aus, bei allerdings erheblich niedrigeren Wirkstoffmengen. Weitere Ergebnisse zu ausgewählten Nitrifikationsinhibitoren sind in Tabelle 5 dargestellt.

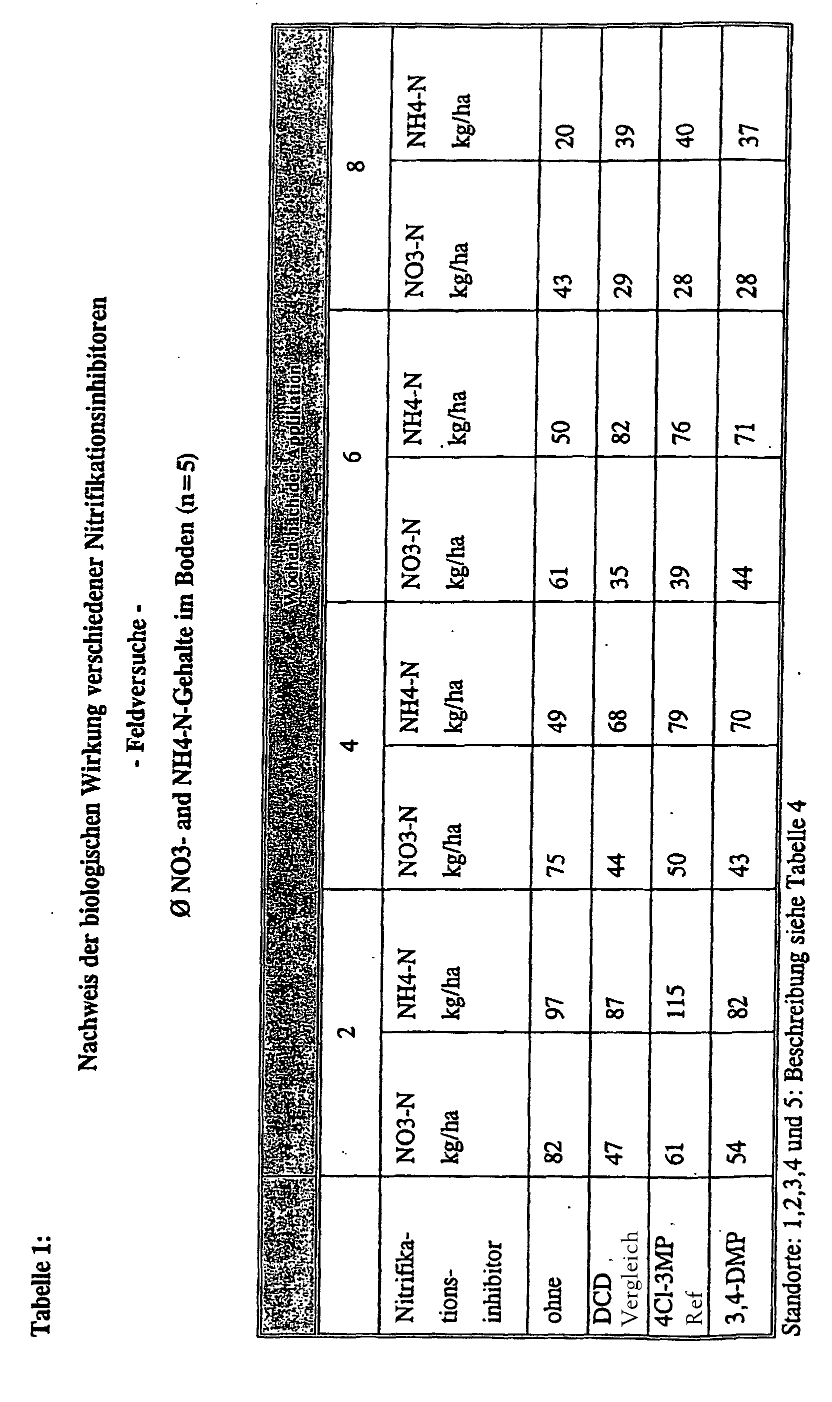

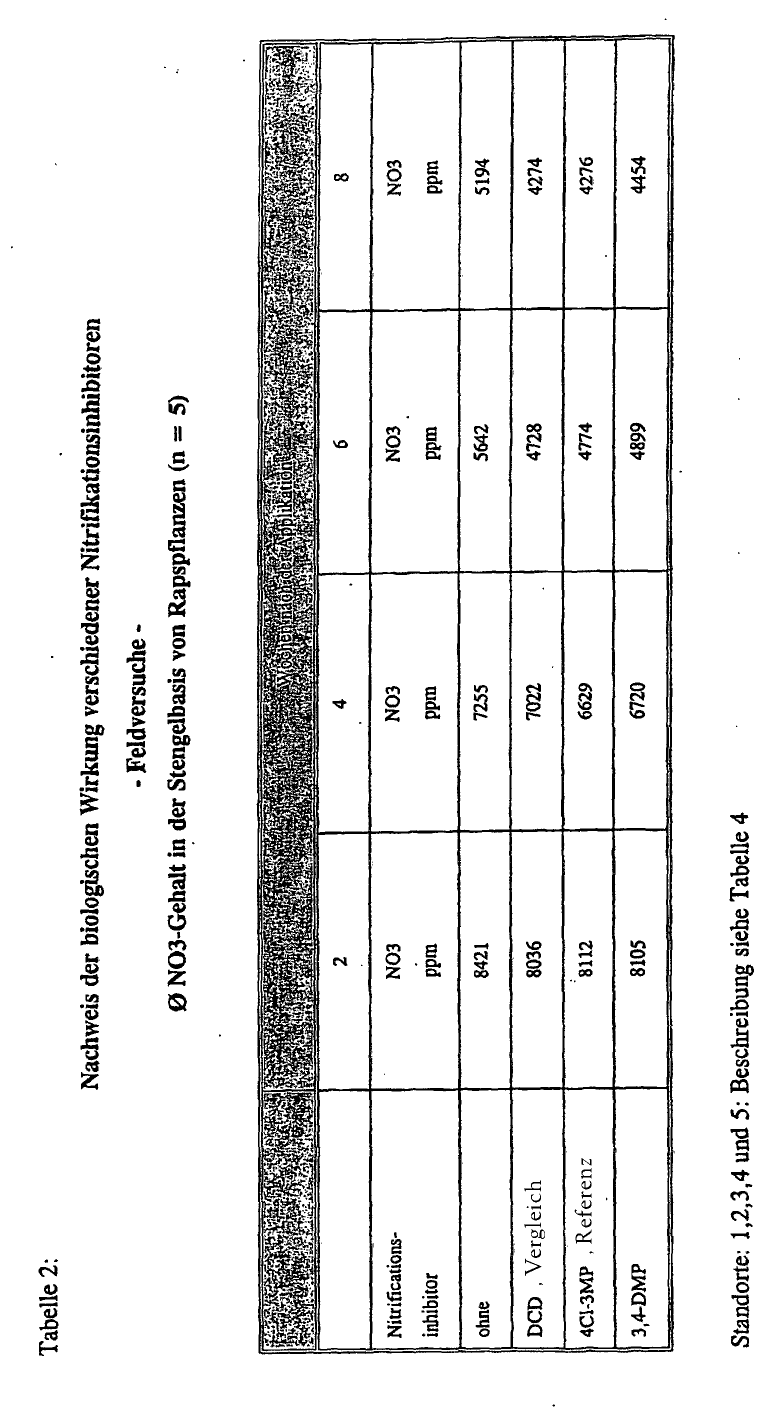

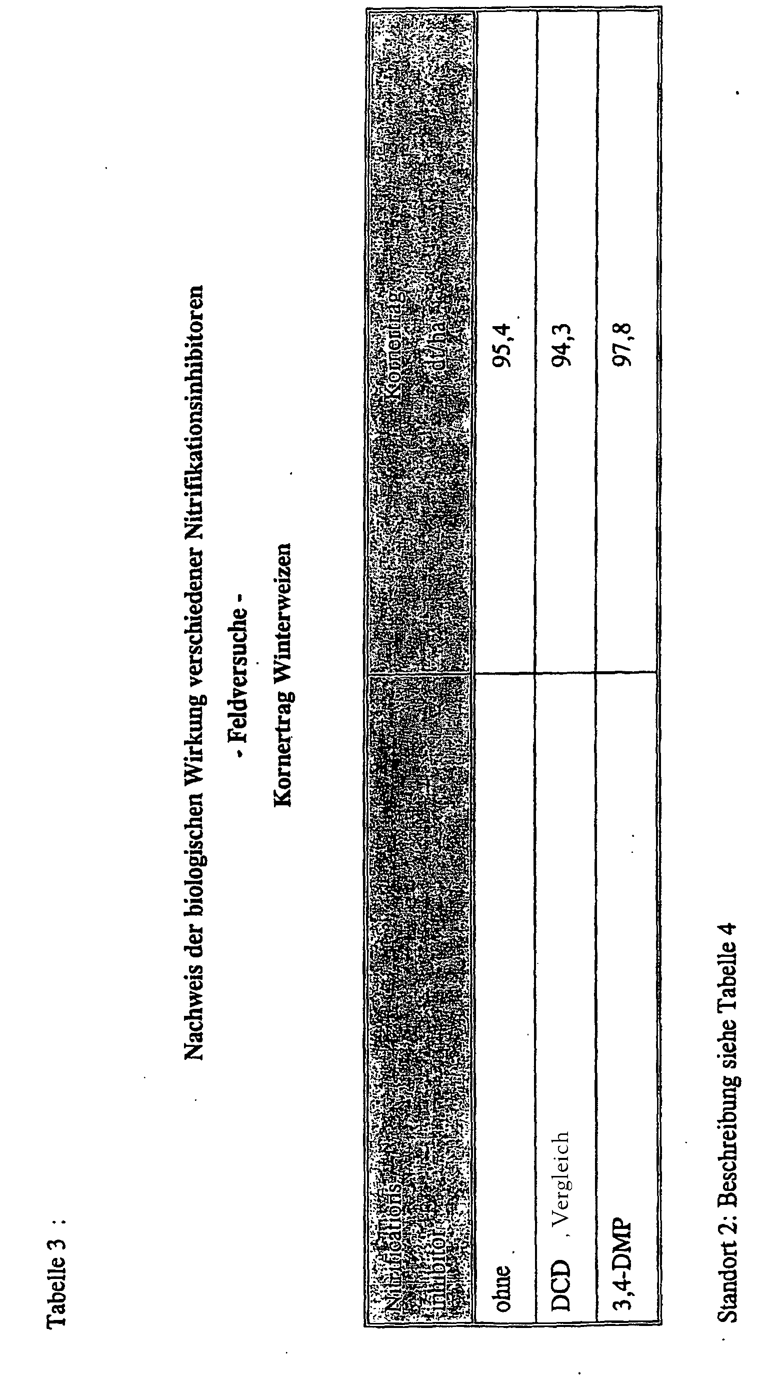

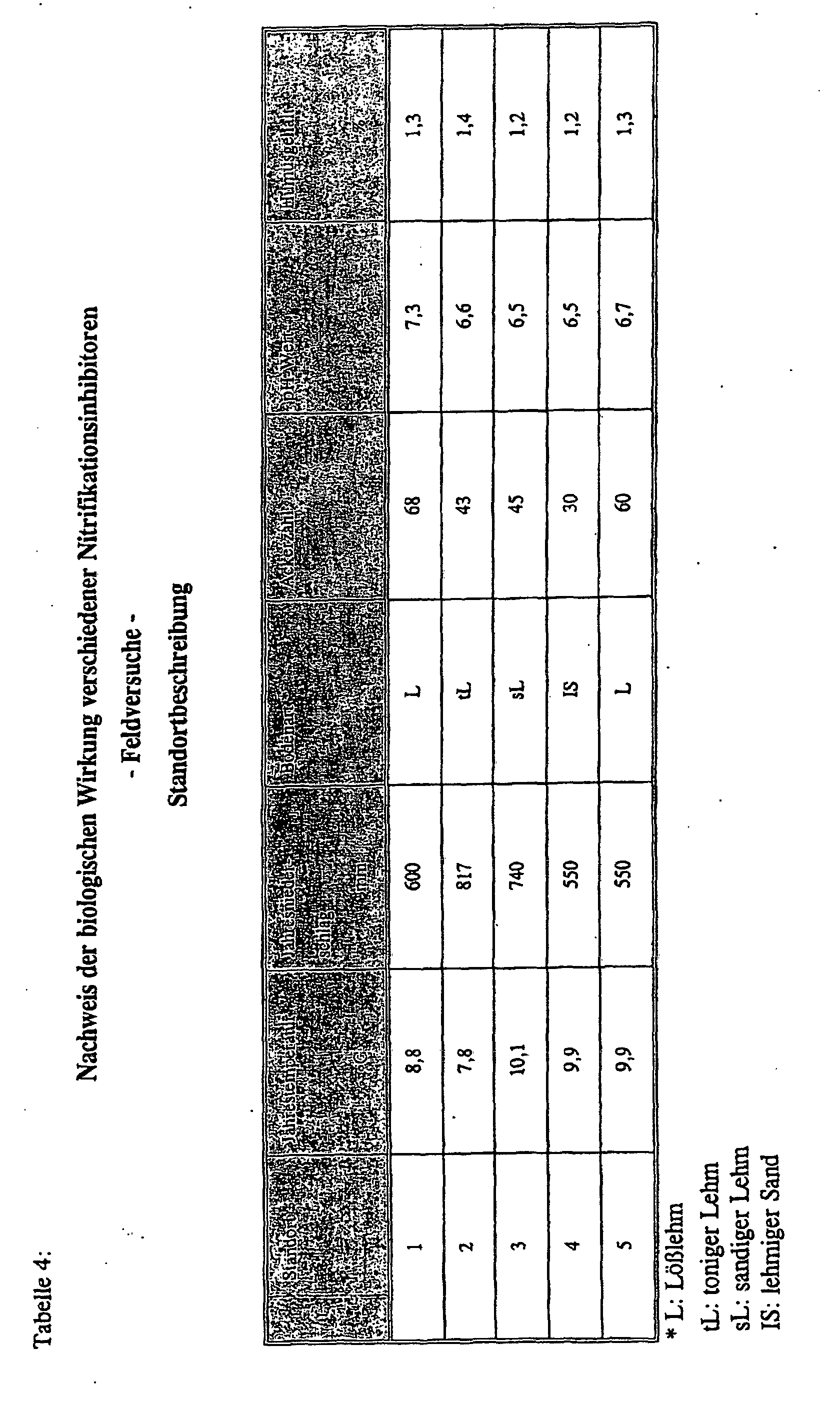

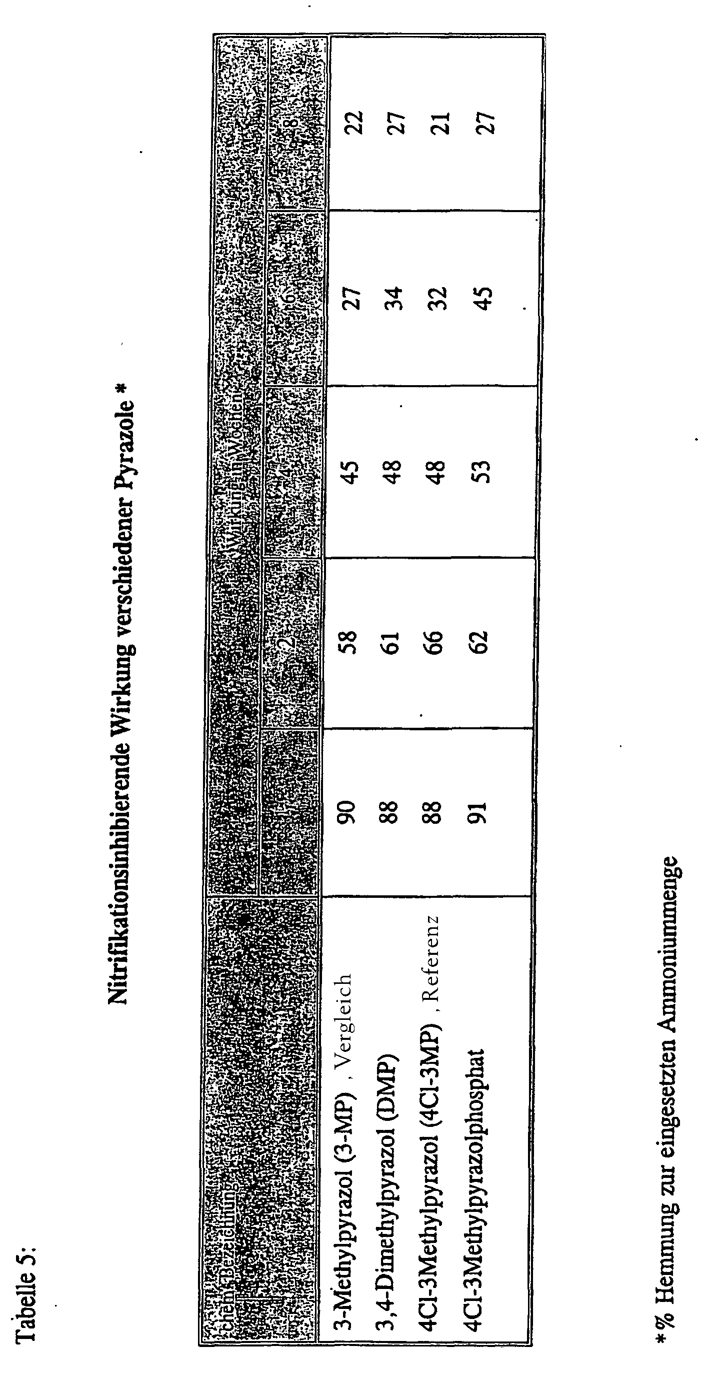

## Patentansprüche

1. Verwendung von 3,4-Dimethylpyrazol oder einem Phosphorsäure-Additionssalz davon oder einem Phosphorsäureadditionssalz von 4-Chlor-3-methylpyrazol als Nitrifikationsinhibitoren.

2. Mineraldüngemittel, enthaltend mindestens eine Verbindung, wie sie in Anspruch 1 definiert ist.

3. Düngeverfahren, **dadurch gekennzeichnet, daß** man Mineraldüngemittel, wie sie in Anspruch 2 definiert sind, auf Ackerboden ausbringt.

4. Verwendung von Verbindungen, wie sie in Anspruch 1 definiert sind, zur Stabilisierung von Gülle oder flüssigen Düngeformulierungen.

5. Phosphorsäure-Additionssalz von 3,4-Dimethylpyrazol, Phosphorsäure-Additionssalz von 4-Chlor-3-methylpyrazol sowie Gemische aus diesen Verbindungen

## Claims

1. The use of 3,4-dimethylpyrazole or of a phosphoric acid addition salt thereof or of a phosphoric acid addition salt of 4-chloro-3-methylpyrazole as nitrification inhibitors.

2. An inorganic fertilizer containing at least one compound as defined in claim 1.

3. A fertilization process wherein mineral fertilizers as defined in claim 2 are applied to arable soil.

4. The use of compounds as defined in claim 1 for stabilizing slurry or liquid fertilizing formulations.

5. A phosphoric acid addition salt of 3,4-dimethylpyrazole, phosphoric acid addition salt of 4-chloro-3-methylpyrazole and also mixtures thereof.

## Revendications

1. Utilisation du 3,4-diméthylpyrazole ou un sel d'addition d'acide phosphorique de celui-ci ou un sel d'addition d'acide phosphorique du 4-chloro-3-méthylpyrazole en tant qu'inhibiteurs de nitrification.

2. Engrais minéraux, contenant au moins un composé tel que défini selon la revendication 1.

3. Procédé de fertilisation, **caractérisé en ce que** l'on applique des engrais minéraux, tels que définis selon la revendication 2, sur une terre arable.

4. Utilisation de composés tels que définis selon la revendication 1, pour stabiliser le purin ou les formulations d'engrais liquides.

5. Sel d'addition d'acide phosphorique du 3,4-diméthylpyrazole, sel d'addition d'acide phosphorique du 4-chloro-3-méthylpyrazole, ainsi que les mélanges de ces composés.
